# FASCICULE DE BREVET EUROPEEN

(11) **EP 1 601 362 B1**
(45) Date de publication et mention de la délivrance du brevet: **15.02.2012**
(21) Numéro de dépôt: 04719486.5
(22) Date de dépôt: 11.03.2004
(51) Int. Cl.: A61K 31/549, A61P 27/02

(54) **UTILISATION DU DIAZOXIDE DANS LE TRAITEMENT DE L'ISCHAEMIE RETINIENNE**
DIAZOXID ZUR BEHANDLUNG DER ISCHAEMIE DER NETZHAUT
USE OF DIAZOXIDE IN THE TREATMENT OF ISCHEMIA RETINAE

(30) Priorité: 11.03.2003 FR 0303011
(43) Date de publication de la demande: 07.12.2005
(73) Titulaire: CENTRE NATIONAL DE LA RECHERCHE SCIENTIFIQUE (CNRS), 75794 Paris Cedex 16 (FR); Universite De Nice-Sophia Antipolis, 06903 Nice Cedex 02 (FR)
(72) Inventeur: LAZDUNSKI, Michel, F-06000 Nice (FR); ETTAICHE, Mohamed, F-06790 Aspremont (FR)
(74) Mandataire: Novagraaf Technologies
(86) Numéro de dépôt international: PCT/FR2004/000584
(87) Numéro de publication internationale: WO 2004/082582

(56) Documents cités:
- EP-A- 0 956 866
- HANKINS M W ET AL: "Consequences of transient retinal hypoxia on rod input to horizontal cells in the rat retina." VISION RESEARCH. ENGLAND MAR 1993, vol. 33, no. 4, mars 1993 (1993-03), pages 429-436, XP009021495 ISSN: 0042-6989
- M ET AL: "New windows on the mechanism of action of KATP channel openers" TRENDS IN PHARMACOLOGICAL SCIENCES, ELSEVIER TRENDS JOURNAL, CAMBRIDGE, GB, vol. 21, no. 11, 1 novembre 2000 (2000-11-01), pages 439-445, XP004220563 ISSN: 0165-6147

## Description

La présente invention concerne l'utilisation du diazoxide (7-chloro-3méthyl-2H-1,2,4-benzothiadiazine-1,1-dioxide) dans le traitement de l'ischémie rétinienne. L'invention concerne, plus particulièrement, l'utilisation d'une composition contenant du diazoxide pour la préparation d'un médicament destiné au traitement et/ou à la prévention d'une ischémie rétinienne et de maladies associées à une ischémie rétinienne.

Les phénomènes conduisant à la mort cellulaire de la rétine, et par voie de conséquences à la perte de la fonction visuelle, sont des phénomènes qui surviennent dans des processus aussi variés que les ischémies et les radiations lumineuses. Le développement chronique de l'état d'ischémie dans des pathologies comme la rétinopathie diabétique, la rétinopathie engendrée par les radiations et les dommages consécutifs à une occlusion veineuse, conduit à une perturbation de la dépolarisation de la membrane cellulaire ce qui, à long terme, entraîne une destruction irréversible de la rétine.

Ainsi, l'ischémie rétinienne s'observe en clinique dans des situations aiguës telles que les occlusions artérielles ou veineuses de la rétine, les contusions oculaires et aussi dans des pathologies chroniques telles que la dégénérescence maculaire liée à l'âge (DMLA), le glaucome, la rétinopathie diabétique, la rétinopathie du prématuré, les maladies inflammatoires et les hémopathies qui conduisent à un dommage rétinien aboutissant même, dans nombre de cas, à une dégénérescence totale de la rétine. Le glaucome et la dégénérescence maculaire liée à l'âge sont devenus les principales causes de la malvoyance dans les pays occidentaux, liées à l'augmentation de l'espérance de vie et constituent donc un véritable problème de santé publique.

De par la fréquence et la sévérité de ces affections oculaires, il existe un réel besoin d'un traitement efficace destiné à traiter et/ou à prévenir les maladies associées à une ischémie rétinienne.

Afin de contrarier les dommages consécutifs à une ischémie-reperfusion rétinienne, plusieurs approches pharmacologiques ont été utilisées sur le plan expérimental. Les piégeurs des radicaux libres (Celeci et al., 2002 ; Szabo et al., 2001), les antagonistes des récepteurs du glutamate (Lagreze et al., 2001) et des récepteurs adrénergiques (Donello et al., 2001 ; Chao et al., 2001) mais également des facteurs neurotrophiques (Fontaine et al., 2002 ; Seigel et al., 2000 ; Cuevas et al., 1998), des bloqueurs de canaux calciques (Osborne et al., 2002) et des inhibiteurs de la libération synaptique du glutamate (Ettaiche et al., 1999) ont été proposés comme agents thérapeutiques pour le traitement de l'ischémie rétinienne. L'utilisation d'un libérateur du glutamate et plus particulièrement du riluzole dans le traitement de l'ischémie rétinienne fait l'objet de la demande de brevet internationale WO 99/42103 publiée le 26 août 1999.

L'ischémie induite par augmentation de la pression oculaire est le modèle le plus fréquemment utilisé pour étudier à la fois les mécanismes et les thérapies potentielles de l'ischémie rétinienne. Le degré de lésion de la rétine est dépendant de l'espèce animale, de la durée et de l'intensité de la pression oculaire imposée. Contrairement au modèle expérimental d'ischémie rétinienne par ligature du nerf optique, cette technique permet, d'une part, d'éviter une hémorragie intraoculaire consécutive au début de la reperfusion et, d'autre part, offre l'avantage de conserver les structures extraoculaires intactes permettant ainsi de réaliser des applications locales de substances pharmacologiques.

Les différentes approches pharmacologiques, validées sur des modèles expérimentaux animaux, se sont heurtées à deux difficultés qui sont, d'une part, l'aspect protecteur mais non thérapeutique de ces molécules (à l'exception du riluzole), d'autre part, leur utilisation clinique qui nécessite une administration dénuée de tout effet secondaire sur les autres organes. Les différentes voies d'administrations utilisées sont l'injection intravitréenne et les voies orale, systémique et locale. L'injection intravitréenne utilisée pour les facteurs de croissance peut aggraver la réaction inflammatoire consécutive à une ischémie mais également induire, au lieu de l'injection, une prolifération cellulaire. La voie orale se heurte au problème de la biodisponibilité du principe actif au niveau du site d'action, l'injection systémique peut induire des effets secondaires sur d'autres organes et enfin l'application locale peut également induire des effets néfastes sur les autres tissus oculaires en particulier la perte de la sensibilité cornéenne et une mydriase. Les études réalisées par les inventeurs ont permis de montrer que ces effets ne sont pas observés après instillation du diazoxide.

Les études des inventeurs ont montré que la rétine est riche en une classe de canaux potassium (Ettaiche et al., 2001) qui relie les propriétés électriques des cellules excitables au métabolisme et plus particulièrement au rapport ATP/ADP (Bernardi et al., 1992). Ce sont les canaux potassium ATP dépendants ou canaux KATP. Par ailleurs l'activation des canaux KATP par des ouvreurs spécifiques protège l'hippocampe dans le système nerveux central contre l'ischémie (Heurteaux et al. ; 1993). C'est dans cette optique que le diazoxide (7-chloro-3methyl-2H-1,2,4-benzothiadiazine-1,1-dioxide), un ouvreur des canaux potassiques ATP dépendants (Ashcroft et al., 2000), a été testé sur un modèle expérimental d'ischémie-reperfusion de la rétine.

Le diazoxide est une substance pharmaceutique développée à l'origine dans le traitement de l'hypertension. Le diazoxide est également connu pour causer une hyperglycémie transitoire due à une diminution de la sécrétion en insuline et à une diminution de l'utilisation périphérique du glucose (Henquin et al., 1982, Diabetes 31 : 776-783). La demande de brevet internationale WO 94/25033 publiée le 10 novembre 1994 décrit une méthode pour traiter un défaut dans le métabolisme du glucose consistant à administrer du diazoxide.

De plus, le diazoxide est une substance liposoluble et de faible poids moléculaire donc susceptible de traverser la barrière cornéenne animale ou humaine. Sa formulation et son conditionnement en collyre sont faciles à réaliser, ce qui constitue un grand avantage d'un point de vue thérapeutique et économique dans le traitement et/ou la prévention des maladies associées à une ischémie rétinienne.

Les travaux des inventeurs ont permis d'identifier une méthode pour prévenir les effets délétères subis par la rétine neurosensorielle à la suite d'une ischémie induite expérimentalement par l'élévation de la pression intraoculaire. Le traitement thérapeutique consiste en l'instillation locale, dans le sac conjonctival, d'une goutte d'une solution ophtalmique dont le principe actif est le diazoxide. Ce traitement empêche la perte de la fonction visuelle en réduisant les effets délétères induits par l'ischémie-reperfusion, dans les couches ganglionnaire, plexiforme interne et nucléaire interne.

L'ischémie rétinienne induite expérimentalement par augmentation de la pression intraoculaire est le modèle le plus couramment utilisé pour le criblage de molécules et l'étude des mécanismes impliqués dans l'ischémie. Ce modèle appliqué au rat, dont la vascularisation de la rétine est identique à celle de l'homme, engendre des lésions localisées, dans une première phase, au niveau des couches des cellules ganglionnaires et de la plexiforme interne. Ces lésions se traduisent par la mort des cellules ganglionnaires et par une réduction importante de l'épaisseur de la couche plexiforme interne. La seconde phase est caractérisée par une réduction de l'épaisseur aussi bien de la couche nucléaire interne que de la couche des cellules photoréceptrices. L'application topique du diazoxide, après la fin de l'ischémie ou plus tardivement (24 heures après le début de la reperfusion), bloque de façon spectaculaire les lésions engendrées par l'ischémie -reperfusion. Cet effet se traduit au niveau de la fonction visuelle par une meilleure récupération de l'amplitude des ondes A et B, conséquence d'une bonne préservation de la cytoarchitecture de la rétine. Ces résultats reportés ici (effet thérapeutique par application locale) désignent le diazoxide comme une molécule à haut potentiel thérapeutique contre l'ischémie rétinienne et les maladies associées à une ischémie rétinienne.

Par conséquent, la présente invention concerne l'utilisation d'une composition pharmaceutique comprenant du diazoxide (7-chloro-3methyl-2H-1,2,4-benzothiadiazine-1,1-dioxide) pour la préparation d'un médicament destiné au traitement et/ou à la prévention d'une maladie associée à l'ischémie rétinienne.

De façon avantageuse, la composition pharmaceutique utilisée dans le cadre de la présente invention comprend entre de 0,001 % à 0,1 % de diazoxide. Dans cette composition, le diazoxide se trouve dans un véhicule pharmaceutiquement applicable. Dans le cadre d'une application oculaire de cette composition, l'homme du métier connaît de tels véhicules pharmaceutiquement acceptables tels que, à titre d'exemple et de façon non exhaustive, du sérum physiologique stérile.

Les rétinopathies diabétiques où celles induites par les radiations et les dommages rétiniens provoqués par une ischémie (phénomène multifactoriel) résultent de mécanismes complexes. Par conséquent, l'association de plusieurs molécules ayant des cibles, et donc des vertus thérapeutiques différentes, devrait permettre un meilleur recouvrement de la fonction rétinienne.

Dans cette optique, le riluzole (2-amino-6-trifluoromethoxy benzothiazole) dont la propriété principale est d'inhiber la libération présynaptique du glutamate (Ettaiche et al., 1999), via l'activation d'autres types de canaux potassiques ; les canaux TREK et TRAAK (Duprat et al., 2000), et dont les effets thérapeutiques par application locale ont été démontrés expérimentalement, serait une molécule de choix à associer au diazoxide dans les pathologies induites par des ischémies rétiniennes.

Par conséquent, la présente invention concerne l'utilisation d'une composition pharmaceutique comprenant, en plus du diazoxide, du riluzole ou un dérivé actif en neuroprotection de celui-ci ou un sel pharmaceutiquement acceptable de celui-ci pour la préparation d'un médicament destiné au traitement et/ou à la prévention d'une maladie associée à l'ischémie rétinienne.

On entend par « dérivé actif en neuroprotection du riluzole » et par « un sel pharmaceutiquement acceptable du riluzole », les molécules décrites dans la demande de brevet internationale WO 99/42103 publiée le 26 août 1999. L'homme du métier est à même de choisir le cas échéant la forme la plus adaptée à la nouvelle application selon l'invention. Comme sels pharmaceutiquement acceptables, on peut citer notamment les sels d'addition avec les acides minéraux tels que chlorhydrate, sulfate, nitrate, phosphate ou organiques tels que acétate, propionate, succinate, oxalate, benzoate, fumarate, maléate, méthanesulfonate, isétionate, etc..., ou des dérivés de substitution de ceux-ci.

Les compositions pharmaceutiques selon l'invention sont constituées par au moins le diaxozide sous forme libre ou sous forme d'un sel d'addition avec un acide pharmaceutiquement acceptable, à l'état pur ou sous forme d'une composition dans laquelle cet actif est associé à tout autre produit pharmaceutiquement compatible. Les médicaments selon l'invention peuvent être employés par voie orale, parentérale, rectale ou topique.

A titre de compositions solides pour administration orale, on peut utiliser des comprimés, des pilules, des poudres, etc... où le diaxozide est mélangé à un ou plusieurs diluants inertes classiquement utilisés, et éventuellement à d'autres substances, tels que par exemple un lubrifiant, un colorant, un enrobage etc....

A titre de compositions liquides pour administration orale ou oculaire, on peut utiliser des suspensions, des solutions, des suspensions, des émulsions, des sirops pharmaceutiquement acceptables contenant des diluants inertes classiquement utilisés, et éventuellement d'autres substances comme des produits mouillants, édulcorants, épaississants, etc.

Les compositions stériles pour administration parentérale peuvent être des solutions aqueuses ou non, des suspensions ou des émulsions. Comme solvant ou véhicule, on peut employer l'eau, le propylène glycol, des huiles végétales ou d'autres solvants organiques convenables. Ces compositions peuvent également contenir des adjuvants, comme des agents mouillants, isotonisants, émulsifiants, etc....

Les compositions pour l'administration topique peuvent être par exemple des crèmes, lotions, collutoires, gouttes nasales ou oculaires ou un aérosol.

Comme cela apparaît des expériences rapportées ci-après, une forme d'application préférée de la composition pharmaceutique utilisée dans le cadre de la présente invention est une application locale dans l'oeil du médicament objet de l'invention. Cette application dans l'oeil est, avantageusement, une instillation locale dans le sac conjonctival. Comme l'application locale dans l'oeil constitue un mode d'utilisation avantageux du diazoxide dans l'application selon l'invention. Ainsi, on envisage tout particulièrement une composition pharmaceutique se présentant sous forme d'un collyre.

L'invention concerne aussi le procédé de préparation des médicaments selon l'invention consistant à mélanger le diazoxide avec éventuellement du riluzole, un dérivé actif en neuroprotection de celui-ci, un sel pharmaceutiquement acceptable de celui-ci, avec un ou plusieurs diluants et/ou adjuvants compatibles et pharmaceutiquement acceptables.

La présente invention concerne également une méthode de traitement et/ou de prévention d'une maladie associée à une ischémie rétinienne consistant à administrer au patient souffrant ou susceptible de souffrir de ladite maladie une quantité suffisante d'une composition pharmaceutique telle que décrite ci-dessus.

Dans le cadre de la présente invention, on entend par « maladie associée à une ischémie rétinienne », une maladie choisie dans le groupe constitué par les occlusions artérielles ou veineuses de la rétine, les contusions oculaires et aussi dans des pathologies chroniques telles que la dégénérescence maculaire liée à l'âge (DMLA), le glaucome, la rétinopathie diabétique, la rétinopathie du prématuré, les maladies inflammatoires et les hémopathies.

D'autres avantages et caractéristiques de l'invention apparaîtront à la lecture des exemples qui suivent concernant l'étude des effets d'un traitement au diazoxide sur des rats ischémiés. Ils font référence aux figures jointes dans lesquelles :

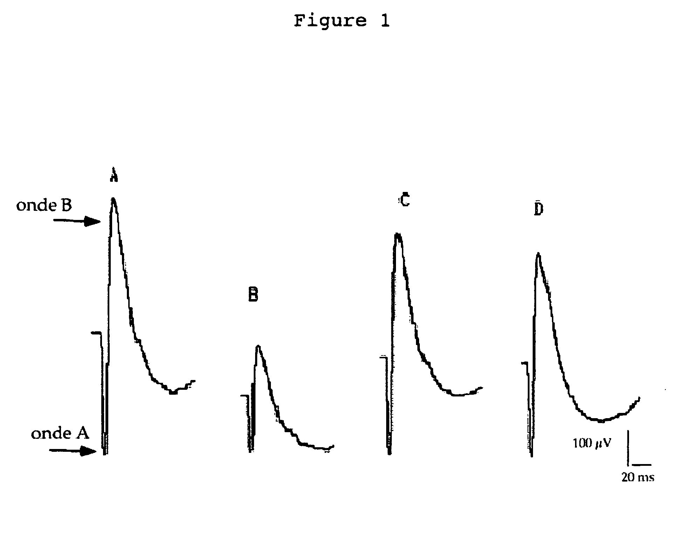

- la figure 1 concerne des profils représentatifs des électrorétinogrammes enregistrés dans les différents groupes testés (A à D) 7 jours après la fin de l'ischémie rétinienne. La figure 1A correspond au groupe contrôle (rats témoins non-ischémiés et non-traités), la figure 1B au groupe de rats ayant subi une ischémie de 40 min mais non-traités, la figure 1C au groupe de rats traités par du diazoxide appliqué 30 min après la fin de l'ischémie et le groupe 1D au groupe de rats traités par du diazoxide appliqué 24 heures après la fin de l'ischémie.

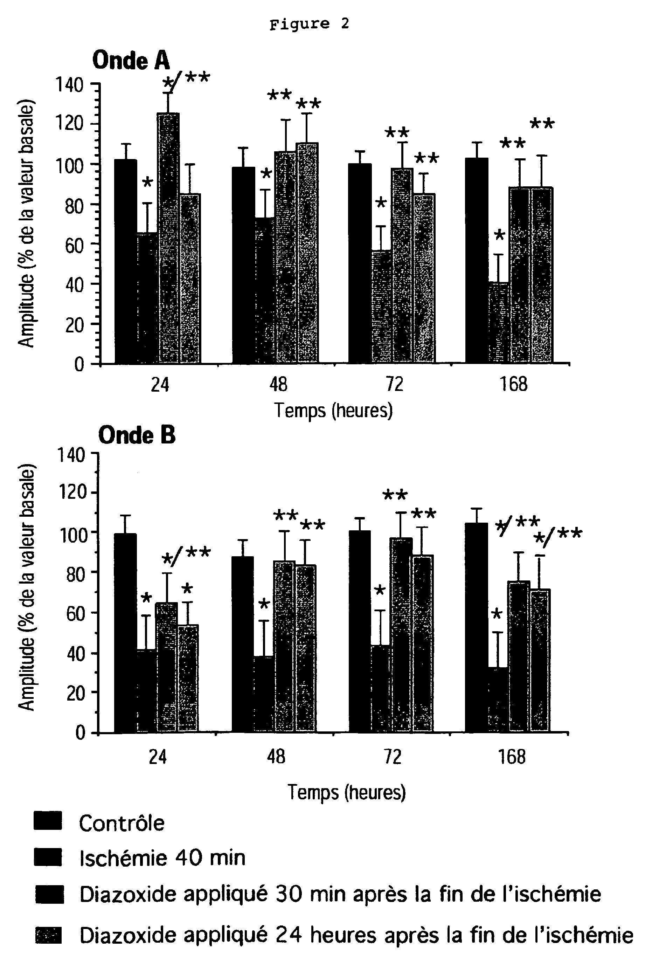

- la figure 2 représente l'amplitude des ondes A (figure 2A) et B (figure 2B) de l'électrorétinigramme au temps 24, 48, 72 et 168 heures pour les différents groupes testés. Les différents groupes testés sont :
   i) le groupe contrôle de rats témoins non-ischémiés et non-traités,
   ii) le groupe de rats ayant subi une ischémie de 40 min mais non-traités,
   iii) le groupe de rats traités par du diazoxide appliqué 30 min après la fin de l'ischémie et
   iv) le groupe de rats traités par du diazoxide appliqué 24 heures après la fin de l'ischémie.
   les valeurs sont exprimées en pourcentage de la valeur basale ± l'écart-type (n = 7 pour chaque histogramme). * indique une différence significative entre le groupe ischémié-non traité ou ischémie-traité au diazoxide et le groupe contrôle. ** indique une différence significative entre le groupe ischémie-non traité ou les groupes ischémies-traités au diazoxide.

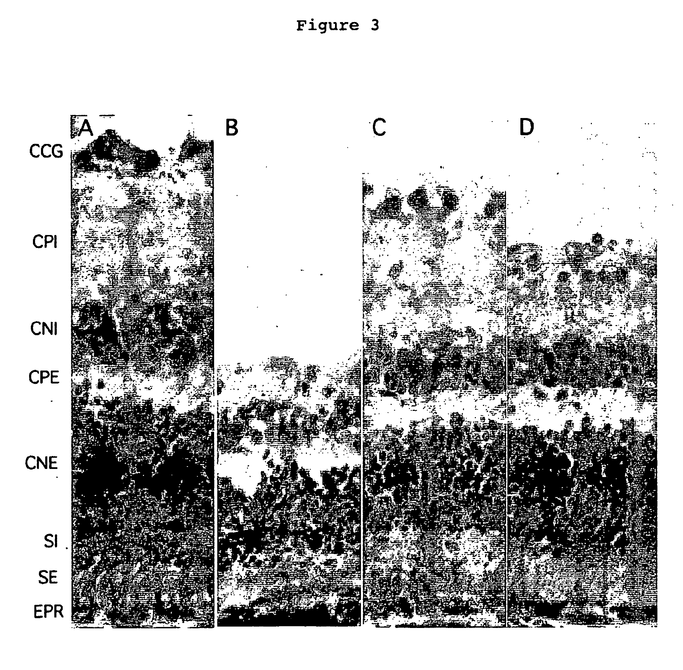

- la figure 3 représente des photomicrographies représentatives de la cyto-architecture de la rétine au temps 7 jours après la fin de l'ischémie dans les différents groupes testés (A à D). La figure 3A correspond au groupe contrôle (rats témoins non-ischémiés et non-traités), la figure 3B au groupe de rats ayant subi une ischémie de 40 min mais non-traités, la figure 3C au groupe de rats traités par du diazoxide appliqué 30 min après la fin de l'ischémie et le groupe 3D au groupe de rats traités par du diazoxide appliqué 24 heures après la fin de l'ischémie. Les abréviations utilisées sont :
   - CCG pour couche des cellules ganglionnaires,
   - CPI pour couche plexiforme interne,
   - CNI pour couche nucléaire interne,
   - CPE pour couche plexiforme externe,
   - CNE pour couche nucléaire externe,
   - SI pour segments internes,
   - SE pour segments externes,
   - EPR pour épithéliums pigmentaire rétinien.

### I. Matériel et méthodes.

### I.1. Animaux.

Des rats Brown Norway en provenance de l'élevage scientifique IFFA CREDO de poids compris entre 200 et 250 g sont réceptionnés et placés dans des cages individuelles dans une salle climatisée (température comprise entre 18 et 20°C) avec un cycle lumière-obscurité (12h/12h). Durant l'étude, les animaux ont libre accès à la nourriture et à l'eau.

### I.2. Administration locale.

Le diazoxide est dissous dans une solution pure de diméthyl-sulfoxide (DMSO). La solution ainsi obtenue, est diluée dans du sérum physiologique stérile afin d'obtenir une solution ophtalmique finale à 0,01% en principe actif et 0,4% en DMSO.

L'administration locale est réalisée par instillation de 10 µl de la solution ophtalmique dans le cul-de-sac conjonctival de l'oeil droit, 30 minutes après à la fin de l'ischémie pour le 1^{er} groupe et 24 heures après le début de la reperfusion pour le second groupe, puis chaque jour pendant 4 jours.

### I.3. Induction de l'ischémie rétinienne.

Après anesthésie générale des rats, par injection intra-péritonéale de pentobarbital sodique (60 mg/kg), la pupille est dilatée par instillation d'une goutte de Mydriaticum®. La chambre antérieure de l'oeil droit, préalablement anesthésiée par instillation d'une goutte d'oxybuprocaïne, est canulée avec une aiguille 30G connectée à un réservoir contenant une solution saline équilibrée de Hank (Hank's Balanced Salt Solution). L'ischémie rétinienne, d'une durée de 40 min, est induite par élévation du réservoir contenant la solution saline à une hauteur de 185 cm permettant de produire une pression intraoculaire de 130 mm Hg. L'ischémie est caractérisée par la disparition du flux sanguin dans les vaisseaux rétiniens, déterminée par ophtalmoscopie directe du fond d'oeil. Les rats sont sacrifiés après une période de reperfusion de 7 jours. Tous les rats qui ont développé une cataracte ou une hémorragie ont été écartés de l'étude.

### II. Modes d'évaluation.

### II.1. Analyse électrophysiologique.

L'électrorétinographie permet l'évaluation de la fonction rétinienne par mesure de l'activité électrique de la rétine et constitue un outil de choix pour le criblage rapide de molécules potentielles, permettant de révéler les substances les plus prometteuses dans le domaine thérapeutique.

L'électrorétinographie est réalisée sous anesthésie générale des rats et après dilatation de la pupille par instillation d'une goutte de Mydriaticum®. L'animal est préalablement adapté pendant 12 heures à l'obscurité, puis installé sur le flanc dans une cage de Faraday. L'électrode de mesure, constituée d'un anneau en chlorure d'argent (Ag/AgCl) est placée sur la cornée de l'oeil droit. L'électrode de référence constituée d'une aiguille en chlorure d'argent est insérée dans l'oreille de l'animal. Toutes ces opérations sont réalisées sous lumière rouge.

Les stimuli lumineux sont produits par un stroboscope placé à 25 cm de l'oeil, dans l'axe visuel. Le flash de lumière blanche d'une durée de 10 µs et d'une intensité de 2,5.10³ cd.s.m⁻² permet une réponse maximale de la rétine. La récupération du signal électrique, son amplification, sa transmission et sa visualisation sur un micro-ordinateur sont gérées par un système compact. Un logiciel intégré permet l'acquisition des données ainsi que la détermination des amplitudes et des temps de latence des différentes ondes de l'électrorétinogramme (ERG). L'ERG chez le rat se compose de deux ondes ; une onde cornéo-négative ou onde A, reflétant l'activité des photorécepteurs suivie d'une onde de polarité positive ou onde B, reflétant l'activité des cellules de Müller.

Pour l'ensemble des groupes, les ERGs seront enregistrés au temps t = 0 avant l'induction de l'ischémie puis aux temps 1, 2, 3, et 7 jours après le début de la reperfusion.

### II.2. Analyse histologique.

Au 7ème jour de reperfusion, les animaux sont sacrifiés par injection intracardiaque d'une overdose de pentobarbital sodique. Les yeux sont énucléés, fixés pendant 2 heures dans une solution de paraformaldéhyde à 4%, puis transférés dans une solution de sucrose à 20% pendant une nuit sous agitation. Les yeux sont ensuite inclus dans du Tissue-Tek, congelés dans l'isopentane refroidi à l'azote liquide, puis stockés à -80°C. Des coupes de 7 µm sont réalisées au cryostat (Leica), montées sur des lames polylysinées et colorées au bleu de toluidine pour une analyse des modifications morphologiques de la rétine.

### II.3. Analyse statistique.

Les amplitudes pré-ischémiques des ondes A et B de l'ERG sont très variables d'un groupe à l'autre mais également à l'intérieur d'un même groupe. La récupération est exprimée en pourcentage de la valeur initiale et la différence d'efficacité entre chaque traitement est analysée, à l'aide du test U de Mann Whitney. Le seuil de significativité est de 0,02.

### III. Résultats.

### III.1. Profils des électrorétinogrammes.

La Figure 1 compare les profils des électrorétinogrammes enregistrés au temps 7 jours après la fin de l'ischémie chez des rats témoins, ischémiés et non traités et ischémiés-traités. Comparativement à l'ERG normal du groupe contrôle (Fig. 1A), on observe une réduction importante de l'onde B mais également de l'onde A dans le groupe ischémié-non traité (Fig. 1B). Cette réduction de l'activité électrique de la rétine est beaucoup moins drastique dans les groupes traités au diazoxide (Fig. 1C-1D).

### II.2. variations de l'amplitude des ondes A et B des électrorétinogrammes.

La Figure 2 montre que les variations de l'amplitude des ondes A et B enregistrée au temps 24, 48, 72 et 168 heures. Chez les rats témoins non-ischémiés, l'amplitude des ondes A et B reste inchangée au cours du temps. Dans les autres groupes de rats ischémiés, on note des variations de l'amplitude des ondes A et B aux différents temps de l'étude.

Ces variations sont caractérisées, dans le groupe ischémié-non traité, par une altération sévère de l'activité électrique de la rétine. Sept jours après le début de la reperfusion, la récupération de l'amplitude des ondes A et B est respectivement égale à 40,55 ± 13,50 et 31,88 ± 18 % des valeurs basales.

A l'inverse, dans le groupe traité au diazoxide à 0.01%, 30 minutes après le début la reperfusion et tous les jours pendant 4 jours, la récupération est pratiquement totale 3 jours après le début de la reperfusion. L'amplitude des ondes A et B est respectivement égale à 97,01 ± 13 et 96,36 ± 11% des valeurs initiales. Cette récupération, décline légèrement pour atteindre au temps 7 jours après la fin de l'ischémie les valeurs respectives de 88,10 ± 14 et 75 ± 14 % des valeurs initiales.

L'application du diazoxide, 24 heures après le début de la reperfusion, ne permet pas une récupération totale de la fonction visuelle de la rétine au temps 3 jours. Néanmoins, elle permet une meilleure récupération de l'activité électrique de la rétine comparativement au groupe ischémié et non traité. Les valeurs de l'amplitude des ondes A et B sont respectivement égales à 84,26 ± 10 et 88,17 ± 14 % au temps 3 jours et à 87,59 ± 16 % et 70,59 ± 16 % au temps 7 jours.

Enfin, comparativement au groupe ischémié-non traité, l'analyse statistique montre que le diazoxide instillé 30 min ou 24 heures après la fin de l'ischémie a un effet hautement significatif sur la récupération de la fonction rétinienne.

### III.3. Cytoarchitecture de la rétine.

La Figure 3 montre la cytoarchitecture de la rétine dans les différents groupes. Comparativement au groupe « contrôle » (Fig. 3A), la rétine du groupe ischémié-non traité montre une atteinte sévère de la couche des cellules ganglionnaires, associée à une mort cellulaire et une réduction pratiquement totale de l'épaisseur de la couche plexiforme interne. La couche nucléaire interne, très sévèrement affectée, est caractérisée par la présence de cellules éparses en état picnotique. Parallèlement la couche des photorécepteurs est caractérisée par une diminution du nombre de rangées de noyaux des cellules photoréceptrices et par une réduction de l'épaisseur des segments internes et externes (Fig. 3B).

Les rétines des groupes traités au diazoxide (Fig. 3C et 3D) montrent une réduction moins sévère de l'épaisseur des couches plexiforme et nucléaire internes mais également une meilleure préservation des couches des cellules ganglionnaires et des cellules photoréceptrices.

### RÉFÉRENCES

Ashcroft FM, Gribble FM. New windows on the mechanism of action of K(ATP) channel openers. Trends Pharmacol Sci. 2000 Nov;21(11):439-45. Review.
Bernardi H, Fosset M, Lazdunski M. ATP/ADP binding sites are present in the sulfonylurea binding protein associated with brain ATP-sensitive K+ channels. Biochemistry. 1992 Jul 14;31(27):6328-32.
Celebi S, Dilsiz N, Yilmaz T, Kukner AS. Effects of melatonin, vitamin E and octreotide on lipid peroxidation during ischemia-reperfusion in the guinea pig retina. Eur J Ophthalmol. 2002 Mar-Apr;12(2):77-83
Chao HM, Osborne NN. Topically applied clonidine protects the rat retina from ischaemia/reperfusion by stimulating alpha(2)-adrenoceptors and not by an action on imidazoline receptors. Brain Res. 2001 Jun 15;904(1):126-36.
Cuevas P, Carceller F, Redondo-Horcajo M, Lozano RM, Gimenez-Gallego G. Systemic administration of acidic fibroblast growth factor ameliorates the ischemic injury of the retina in rats. Neurosci Lett. 1998 Oct 9;255(1):1-4.
Donello JE, Padillo EU, Webster ML, Wheeler LA, Gil DW. alpha(2)-Adrenoceptor agonists inhibit vitreal glutamate and aspartate accumulation and preserve retinal function after transient ischemia. J Pharmacol Exp Ther. 2001 Jan;296(1):216-23.
Duprat F, Lesage F, Patel AJ, Fink M, Romey G, Lazdunski M. The neuroprotective agent riluzole activates the two P domain K(+) channels TREK-1 and TRAAK. Mol Pharmacol. 2000 May;57(5):906-12.
Ettaiche M, Fillacier K, Widmann C, Heurteaux C, Lazdunski M. Riluzole improves functional recovery after ischemia in the rat retina. Invest Ophthalmol Vis Sci. 1999 Mar;40(3):729-36.
Ettaiche M, Heurteaux C, Blondeau N, Borsotto M, Tinel N, Lazdunski M. ATP-sensitive potassium channels (K(ATP)) in retina: a key role for delayed ischemic tolerance. Brain Res. 2001 Jan 26;890(1):118-29.
Fontaine V, Mohand-Said S, Hanoteau N, Fuchs C, Pfizenmaier K, Eisel U. Neurodegenerative and neuroprotective effects of tumor Necrosis factor (TNF) in retinal ischemia: opposite roles of TNF receptor 1 and TNF receptor 2. J Neurosci. 2002 Apr 1;22(7).
Heurteaux C, Bertaina V, Widmann C, Lazdunski M. K+ channel openers prevent global ischemia-induced expression of c-fos, c-jun, heat shock protein, and amyloid beta-protein precursor genes and neuronal death in rat hippocampus. Proc Natl Acad Sci U S A. 1993 Oct 15;90(20):9431-5.
Osborne NN, Wood JP, Cupido A, Melena J, Chidlow G. Topical flunarizine reduces IOP and protects the retina against ischemia-excitotoxicity. Invest Ophthalmol Vis Sci. 2002 May;43(5):1456-641.
Seigel GM, Chiu L, Paxhia A. Inhibition of neuroretinal cell death by insulin-like growth factor-1 and its analogs. Mol Vis. 2000 Aug 31;6:157-63.
Szabo ME, Haines D, Garay E, Chiavaroli C, Farine JC, Hannaert P, Berta A, Garay RPL. Antioxidant properties of calcium dobesilate in ischemic/reperfused diabetic rat retina. Eur J Pharmacol. 2001 Oct 5;428(2):277-86.
Lagreze WA, Muller-Velten R, Feuerstein TJ. The neuroprotective properties of gabapentin-lactam. Graefes Arch Clin Exp Ophthalmol. 2001 Nov;239(11):845-9.

## Revendications

1. Utilisation du diazoxide (7-chloro-3methyl-2H-1, 2,4-benzothiadiazine-I, I-dioxide) pour la préparation d'un médicament destiné au traitement et/ou à la prévention de l'ischémie rétinienne et/ou des lésions engendrées par l'ischémie rétinienne.

2. Utilisation selon la revendication 1, dans laquelle ledit médicament comprend entre de 0,001 % à 0,1 % de diazoxide.

3. Utilisation selon l'une quelconque des revendications 1 ou 2, dans laquelle ledit médicament comprend, en outre, du riluzole ou un dérivé actif en neuroprotection de celui-ci ou un sel pharmaceutiquement acceptable de celui-ci.

4. Utilisation selon l'une quelconque des revendications 1 à 3, dans laquelle ledit médicament est un médicament à application locale dans l'oeil.

5. Utilisation selon l'une quelconque des revendications 1 à 4, dans laquelle ledit médicament se présente sous la forme d'un collyre.

6. Utilisation selon l'une quelconque des revendications 1 à 4, dans laquelle l'ischémie rétinienne est associée à une maladie choisie dans le groupe constitué par les occlusions artérielles ou veineuses de la rétine, les contusions oculaires et aussi dans des pathologies chroniques telles que la dégénérescence maculaire liée à l'âge (DMLA), le glaucome, la rétinopathie diabétique, la rétinopathie du prématuré, les maladies inflammatoires et les hémopathies.

## Claims

1. Use of diazoxide (7-chloro-3-methyl-2H-1,2,4-benzothiadiazine 1,1-dioxide) in the preparation of a medicament intended for the treatment and/or prevention of retinal ischaemia and/or lesions resulting from retinal ischaemia.

2. Use according to Claim 1, in which the said medicament comprises from 0.001 to 0.1% of diazoxide.

3. Use according to either one of Claims 1 and 2, in which the said medicament additionally comprises riluzole or a derivative which is active in neuroprotection of the latter or a pharmaceutically acceptable salt of the latter.

4. Use according to any one of Claims 1 to 3, in which the said medicament is a medicament for local application in the eye.

5. Use according to any one of Claims 1 to 4, in which the said medicament is provided in the form of an eye lotion.

6. Use according to any one of Claims 1 to 4, in which the retinal ischaemia is associated with a disease chosen from the group consisting of retinal artery or vein occlusions, ocular contusions and also from chronic pathologies, such as age-related macular degeneration (ARMD), glaucoma, diabetic retinopathy, retinopathy of prematurity, inflammatory diseases and haemopathies.

## Patentansprüche

1. Verwendung von Diazoxid (7-Chlor-3-methyl-2H-1,2,4-benzothiadiazin-1,1-dioxid) zur Herstellung eines Medikaments, das zur Behandlung und/oder Vorbeugung von Retinaischämie und/oder der Läsionen bestimmt ist, die von Retinaischämie hervorgerufen werden.

2. Verwendung nach Anspruch 1, wobei das Medikament 0,001 % bis 0,1 Diazoxid umfasst.

3. Verwendung nach einem der Ansprüche 1 oder 2, wobei das Medikament ferner Riluzol oder ein Derivat davon, das bei der Neuroprotektion aktiv ist, oder ein pharmazeutisch verträgliches Salz davon umfasst.

4. Verwendung nach einem der Ansprüche 1 bis 3, wobei das Medikament ein Medikament zur topischen Applikation im Auge ist.

5. Verwendung nach einem der Ansprüche 1 bis 4, wobei das Medikament die Form eines Collyriums aufweist.

6. Verwendung nach einem der Ansprüche 1 bis 4, wobei die Retinaischämie mit einer Krankheit assoziiert ist, die aus der Gruppe bestehend aus Retinavenen- oder Retinaarterienverschlüssen, Augenprellungen und auch chronischen Pathologien, wie etwa altersbedingter Makuladegeneration (AMD), Glaukom, diabetischer Retinopathie, Frühgeborenenretinopathie, entzündlichen Krankheiten und Hämopathien ausgewählt ist.
